# EUROPEAN PATENT APPLICATION

(11) **EP 2 266 541 A1**
(43) Date of publication of application: **29.12.2010**
(21) Application number: 09163177.0
(22) Date of filing: 18.06.2009
(51) Int. Cl.: A61K 9/20, A61K 31/5377

(54) **Solid pharmaceutical composition comprising rivaroxaban**

(71) Applicant: Krka Tovarna Zdravil, D.D., Novo Mesto, 8501 Novo Mesto (SI)
(72) Inventor: Sedmak, Gregor, 1000, Ljubljana (SI); Vrecer, Franc, 8351, Straza pri Novem mestu (SI); Meznar, Klavdija, 8000, Novo mesto (SI); Trost, Sabina, 1330, Kocevje (SI); Bukovec, Polona, 8000, Novo mesto (SI); Hvalec, Miran, 8000, Novo mesto (SI)
(74) Representative: UEXKÜLL & STOLBERG

(57) **Abstract**

The invention relates to a solid pharmaceutical composition comprising rivaroxaban, or a pharmaceutically acceptable salt or solvate thereof, and at least one pharmaceutically acceptable excipient. The invention also relates to a process for the preparation of a solid pharmaceutical composition comprising rivaroxaban, or a pharmaceutically acceptable salt or solvate thereof, the process comprising (a) providing a mixture comprising rivaroxaban and at least one pharmaceutically acceptable excipient and (b) converting said mixture to a solid pharmaceutical composition.

## Description

The invention relates to a solid pharmaceutical composition comprising rivaroxaban. It further relates to a process for the preparation of a pharmaceutical composition comprising rivaroxaban.

### Background of the invention

Rivaroxaban is the INN of the anticoagulant compound 5-chloro-N-{[(5S)-2-oxo-3-[4-(3-oxomorpholin-4-yl)phenyl]oxazolidin-5-yl]methyl}thiophene-2-carboxamide, which was originally disclosed in WO 01/47919 A1. Rivaroxaban is a small molecule inhibitor of blood coagulation factor Xa and is used in the prophylaxis and treatment of thromboembolic diseases such as heart attack, angina pectoris, reocclusion and restenosis following angioplasty or bypass, cerebral apoplexy, transient ischemic attack, peripheral arterial obstructive diseases, pulmonary embolism and venous thrombosis.

Rivaroxaban is characterized by a very low solubility in water (about 7 mg/L) and many organic solvents as well as a high melting point (about 230 °C). The low solubility is particularly problematic with respect to an oral application, which results in a poor bioavailability.

A number of approaches have been pursued in order to increase the oral bioavailability of rivaroxaban.

WO 2005/060940 A2 describes a process for the preparation of a solid pharmaceutical composition comprising rivaroxaban in hydrophilized form, wherein a granulate prepared by moist granulation of rivaroxaban with a hydrophilic binder is converted to the pharmaceutical composition. Tablets comprising hydrophilized active agent are shown to exhibit increased bioavailability as compared with tablets comprising non-hydrophilized active agent. However, the moist granulation processes used for hydrophilization of the active agent are complex, require special equipment and are difficult to use on an industrial scale. Moreover, they require great amounts of energy for the evaporation of granulation liquid.

WO 2007/039122 A2 discloses solid pharmaceutical dosage forms comprising rivaroxaban in amorphous form and/or in the form of thermodynamically metastable crystal modifications. These dosage forms are described as showing an increased rate of dissolution as well as increased solubility. However, obtaining rivaroxaban in amorphous form or in the form of thermodynamically metastable crystal modifications is problematic because it involves dissolving and/or melting the active agent. In particular, dissolution processes require excessively large amounts of solvent because of the poor solubility of rivaroxaban in water and pharmaceutically acceptable organic solvents like ethanol or acetone. Due to the high melting point of rivaroxaban, processes involving melting the active agent result in significant decomposition thereof.

WO 2007/039132 A1 discloses certain polymorphous forms as well as the amorphous form of rivaroxaban.

It is an object of the present invention to provide a pharmaceutical composition comprising rivaroxaban avoiding these drawbacks and which is not only bioequivalent with the known formulations of rivaroxaban, but which can also be prepared from inexpensive excipients and can be prepared using a simple and economical process that is better suited for industrial application.

### Description of the invention

In one aspect the invention relates to a solid pharmaceutical composition comprising rivaroxaban, or a pharmaceutically acceptable salt or solvate thereof, and at least one pharmaceutically acceptable excipient. It is preferred that the solid pharmaceutical composition is in the form of a tablet or capsule or in the form of pellets or granules.

The composition according to the invention comprises rivaroxaban, or a pharmaceutically acceptable salt or solvate thereof, as active ingredient. Generally, rivaroxaban can be used in any crystalline, partly crystalline or amorphous form or modification. Preferably, rivaroxaban is used in the crystalline modification in which it is obtained according to the processes disclosed in WO 01/47919 A1, J. Med. Chem 48, 5900-5908 (2005) or WO 2007/039132. It is further preferred that the rivaroxaban is in non-hydrophilized form.

Preferably the rivaroxaban is in micronized form with d₉₀ < 60 µm, more preferably d₉₀ < 40 µm, and most preferably d₉₀ < 30 µm. As used herein d₉₀ < x means that at least 90 % by volume of the particles have a particle size below x. The particle size can be determined by laser light scattering for instance using a Malvern Mastersizer Apparatus MS 2000 equipped with a Hydro S dispersion unit using purified water as the dilution medium. Micronized rivaroxaban can be obtained for instance by single or multi-stage micronization in the dry state using dry mills, such as cutting mills, pin/cage mills, hammer mills, jet mills, fluidized bed jet mills, ball mills and roller mills.

The composition preferably comprises 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof. All percentages given herein are by weight of the total composition unless specifically stated otherwise.

The term "pharmaceutical acceptable excipient" as used herein refers to additives useful for converting pharmacologically active compounds into pharmaceutical dosage forms which are suitable for administration to patients. Suitable excipients include fillers, binders, disintegrants, surfactants, lubricants, glidants and coloring agents. Other pharmaceutically acceptable excipients can also be included.

The composition according to the invention typically comprises at least one filler. Suitable fillers include monosaccharides and oligosaccharides such as glucose, fructose, saccharose, lactose (anhydrous and monohydrate), raffinose, trehalose and dextrates, sugar alcohols such as mannitol, sorbitol, maltitol, xylitol and lactitol, compressible sugar, microcrystalline cellulose, powdered cellulose, siliconized microcrystalline cellulose, calcium hydrogen phosphate, calcium carbonate, calcium lactate and mixtures thereof. Microcrystalline cellulose and water-soluble fillers are preferred. Water-soluble fillers are particularly preferred.

The composition preferably comprises 20 to 99 wt.-%, particularly 50 to 95 wt.-%, more preferably 70 to 90 wt.-%, most preferably 80 to 90 wt.-% of filler. According to another embodiment, the composition comprises 20 to 99 wt.-%, particularly 30 to 90 wt.-%, more preferably 40 to 70 wt.-% and most preferably 50 to 60 wt.-% of filler.

The term "water-soluble filler" as used herein refers to a filler having a solubility in water at 25 °C of at least 0.03 g/L. Examples of water-soluble fillers include monosaccharides and oligosaccharides such as glucose, fructose, saccharose, lactose (anhydrous and monohydrate), raffinose, trehalose and dextrates, sugar alcohols such as mannitol, sorbitol, maltitol, xylitol and Lactitol. Lactose, mannitol, sorbitol or xylitol are particularly preferred. It is particularly preferred that the composition comprises at least 35 wt.-%, particularly 40 to 70 wt.-%, more preferably 40 to 60 wt.-% and most preferably 45 to 55 wt.-% of at least one water-soluble filler.

The composition can also include at least one water-insoluble filler. The term "water-insoluble filler" as used herein refers to a filler having a solubility in water at 25 °C of less than 0.03 g/L. Examples of water-insoluble fillers include microcrystalline cellulose, powdered cellulose, siliconized microcrystalline cellulose, calcium hydrogen phosphate, calcium carbonate, calcium lactate and mixtures thereof. Microcrystalline cellulose and calcium hydrogen phosphate are particularly preferred. It is preferred that the composition comprises 5 to 50 wt.-%, more preferably 10 to 40 wt.-% and most preferably 30 to 40 wt.-% of at least one water-insoluble filler. According to another embodiment, the composition comprises 5 to 50 wt.-%, more preferably 8 to 30 wt.-% and most preferably 10 to 20 wt.-% of at least one water-insoluble filler.

It is further preferred that the composition comprises water-soluble filler and water-insoluble filler in a weight ratio of 1:1 to 10:1, particularly 1:1 to 5:1, and most preferably 1.1:1 to 1.5:1. According to another embodiment, the filler comprises water-soluble filler and water-insoluble filler in a weight ratio of 1:1 to 10:1, particularly 2:1 to 8:1, and most preferably 3:1 to 5:1.

Suitable binders include povidone (polyvinylpyrrolidone), copovidone (vinylpyrrolidone-vinylacetate copolymer), microcrystalline cellulose, powdered cellulose, crystalline cellulose, siliconized microcrystalline cellulose, cellulose derivatives such as hydroxymethylcellulose, hydroxyethylcellulose, hydroxypropylcellulose and hydroxypropylmethylcellulose, low-substituted hydroxypropylcellulose, starch, pregelatinized starch, polymethacrylates, compressible sugars, sucrose and sugar alcohols such as mannitol, sorbitol, maltitol and xylitol and mixtures thereof. Preferred are povidone, copovidone, mannitol and sorbitol.

According to a preferred embodiment, the binder comprises at least one low-melting binder having a melting point below 120 °C, particularly below 100 °C and most preferably below 80 °C. Suitable low-melting binders include poloxamers, polyethyleneglycols having an average molecular weight of below 15,000 and particularly in the range of 1,500 to 10,000, macrogol glycerides such as Gelucire^{®} and esters of glycerol with fatty acids having 10 to 24 carbon atoms such as glycerol monostearate and glycerol behenate. Binders having a melting point below 80°C and/or having surfactant properties are particularly preferred. As used herein, the term "having surfactant properties" refers to a substance decreasing the surface tension of water by at least 20 % in comparison to pure water at 25 °C when used at a concentration of 0.1 g/L or less. Low temperature-melting binders and binders having surfactant properties are particularly suitable for use in hot melt processes.

The composition preferably comprises 0.5 to 40 wt.-%, particularly 1 to 20 wt.-%, more preferably 2 to 10 wt.-%, most preferably 3 to 7 wt.-% of binder. According to another embodiment, the composition comprises 0 to 40 wt.-%, particularly 1 to 30 wt.-%, more preferably 5 to 25 wt.-%, and most preferably 10 to 20 wt.-% of low temperature-melting binder.

The term "disintegrant" as used herein refers to any material that has wicking and/or swelling properties when it comes in contact with water. Suitable disintegrants include povidone, crospovidone, starch, pregelatinized starch, sodium starch glycolate, hydroxypropyl starch, microcrystalline cellulose, carboxymethylcellulose sodium or calcium, croscarmellose, polacrilin potassium, low-substituted hydroxypropylcellulose, sodium or calcium alginate, docusate sodium, methylcellulose, agar, guar gum, chitosan, alginic acid and mixtures thereof. Preferred are crospovidone and croscarmellose. The composition preferably comprises 1 to 20 wt.-%, particularly 2 to 10 wt.-%, more preferably 3 to 8 wt.-%, and most preferably 4 to 7 wt.-% of disintegrant.

Suitable surfactants include anionic surfactants such as sodium lauryl sulfate, cationic surfactants such as cetrimide, ampholytic surfactants such as N-dodecyl-N,N-dimethylbetaine, non-ionic surfactants such as sorbitan fatty acid esters (e.g. Spans^{®}), polysorbates (e.g. Tweens^{®}), polyoxyethylene alkyl Ethers (e.g. Brij^{®}), poloxamers (e.g. Lutrol^{®}) and mixtures thereof. Preferred surfactants include sodium lauryl sulfate, polysorbates and poloxamers. Sodium lauryl sulfate is particularly preferred. The composition preferably comprises 0.5 to 30 wt.-%, particularly 1 to 10 wt.-%, more preferably 1.5 to 5 wt.-%, and most preferably 2 to 3 wt.-% of surfactant. According to another embodiment, the composition comprises 0 to 30 wt.-%, particularly 0.2 to 5 wt.-%, more preferably 0.5 to 3 wt.-%, and most preferably 1 to 2 wt.-% of surfactant.

Suitable lubricants include fatty acids such as stearic acid, palmitic acid and oleic acid, fatty acid salts such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. The composition preferably comprises 0.1 to 10 wt.-%, particularly 0.25 to 5 wt.-%, and more preferably 0.5 to 2 wt.-% of lubricant.

Suitable glidants include colloidal silicon dioxide and magnesium trisilicate. The composition preferably comprises 0.1 to 10 wt.-%, particularly 0.25 to 5 wt.-%, and more preferably 0.5 to 2 wt.-% of glidant.

Suitable coloring agents include dyes and pigments such as iron oxide and titanium oxide. The composition preferably comprises 0.001 to 1 wt.-%, particularly 0.01 to 0.5 wt.-%, and more preferably 0.05 to 0.2 wt.-% of coloring agent.

The composition can also include an acid which may serve to improve the solubility of the active ingredient. Preferred acids are organic acids, in particular citric acid. The acid is preferably present in an amount of 0 to 15 wt.-%, in particular 1 to 12 wt.-%.

According to a preferred embodiment, the composition comprises
(a) 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof;
(b) 30 to 99 wt.-%, particularly 50 to 95 wt.-%, more preferably 70 to 90 wt.-%, most preferably 80 to 87 wt.-% of filler;
(c) 0 to 40 wt.-%, particularly 1 to 20 wt.-%, more preferably 2 to 10 wt.-%, most preferably 3 to 7 wt.-% of binder;
(d) 0 to 20 wt.-%, particularly 2 to 10 wt.-%, more preferably 3 to 8 wt.-%, most preferably 4 to 7 wt.-% of disintegrant;
(e) 0 to 30 wt.-%, particularly 1 to 10 wt.-%, more preferably 1.5 to 5 wt.-%, most preferably 2 to 3 wt.-% of surfactant;
(f) 0 to 10 wt.-%, particularly 0.25 to 5 wt.-%, more preferably 0.5 to 2 wt.-% of lubricant;
(g) 0 to 10 wt.-%, particularly 0.25 to 5 wt.-%, more preferably 0.5 to 2 wt.-% of glidant;
(h) 0 to 1 wt.-%, particularly 0.01 to 0.5 wt.-%, more preferably 0.05 to 0.2 wt.-% of coloring agent; and
(i) 0 to 15 wt.-%, and particularly 1 to 12 wt.-% of organic acid.

Compositions according to this embodiment are particularly suitable to be prepared by using dry mixing, dry granulation or co-milling.

According to another preferred embodiment, the composition comprises
(a) 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof;
(b) 20 to 99 wt.-%, particularly 30 to 90 wt.-%, more preferably 40 to 70 wt.-%, most preferably 50 to 60 wt.-% of filler;
(c) 0 to 40 wt.-%, particularly 1 to 30 wt.-%, more preferably 5 to 25 wt.-%, most preferably 10 to 20 wt.-% of binder;
(d) 0 to 20 wt.-%, particularly 2 to 10 wt.-%, more preferably 3 to 8 wt.-%, most preferably 4 to 7 wt.-% of disintegrant;
(e) 0 to 30 wt.-%, particularly 0.2 to 5 wt.-%, more preferably 0.5 to 3 wt.-%, most preferably 1 to 2 wt.-% of surfactant;
(f) 0 to 10 wt.-%, particularly 0.25 to 5 wt.-%, more preferably 0.5 to 2 wt.-% of lubricant;
(g) 0 to 10 wt.-%, particularly 0.25 to 5 wt.-%, more preferably 0.5 to 2 wt.-% of glidant;
(h) 0 to 1 wt.-%, particularly 0.01 to 0.5 wt.-%, more preferably 0.05 to 0.2 wt.-% of coloring agent;
(i) 0 to 15 wt.-%, and particularly 1 to 12 wt.-% of organic acid.

Compositions according to this embodiment are particularly suitable to be prepared by using hot melt processes such as hot melt granulation or hot melt extrusion.

The composition is optionally film coated. Film coating can be used to provide for improved surface smoothness and color, increased chemical and physical stability of the active agent due to reduced permeability for gases such as oxygen and/or water vapor, less disintegration of the solid composition in acidic medium resulting in decreased gastrointestinal side effects, and easier swallowing of tablet. The film coating comprises coating materials generally known in the art. Suitable coating materials include polymers such as hydroxypropylcellulose, hydroxypropylmethylcellulose, ethylcellulose, povidone and copovidone, graft copolymers of polyethyleneglycol and polyvinyl alcohol (Kollicoat IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyl citrate, antitacking agents such as talc, glycerol monostearate, magnesium stearate and colorants or pigments such as titanium dioxide or iron oxide.

According to one embodiment, the composition according to the invention exhibits a release profile **characterized in that** at least 40 %, particularly at least 50 %, more preferably at least 60 %, most preferably at least 70 % by weight of the active agent are released within a period of 30 minutes under non-sink conditions (USP paddle, 75 rpm, 900 mL acetate buffer pH 4.5).

According to another embodiment, the composition according to the invention exhibits a release profile **characterized in that** at least 80 %, particularly at least 90 %, more preferably at least 95 %, most preferably at least 97 % by weight of the active agent are released within a period of 30 minutes under sink conditions (USP paddle, 75 rpm, 900 mL acetate buffer pH 4.5 containing 0.5 % sodium lauryl sulfate).

It has surprisingly been found that the solid pharmaceutical composition according to the invention exhibits a dissolution profile and bioavailability that is at least comparable to that of known rivaroxaban formulations, particularly formulations comprising rivaroxaban in hydrophilized form, while allowing for substantially less complex and expensive preparation.

Another advantage is that the solid pharmaceutical composition according to the invention can be prepared in the absence of solvent, which results in a lower consumption of energy and which is more environmentally friendly.

The composition according to the invention can be packaged into various kinds of containers. Thus, the invention also relates to a packaged pharmaceutical composition comprising the solid pharmaceutical composition described above packaged in a container made of glass or polypropylene with or without desiccant or in a blister made of single or multiple polymer and/or aluminium layers. According to a particular embodiment, the packaged composition is packaged with an atmosphere having a reduced oxygen concentration such as below 15 vol.-%, particularly below 10 vol.-%, and more preferably below 5 vol.-% oxygen. According to a particularly preferred embodiment, the composition is packaged with a nitrogen atmosphere.

The composition according to the invention can be prepared in principle by any method used for manufacturing solid pharmaceutical compositions. Preferred processes for the preparation of the composition are as described below.

The invention also relates to a process for the preparation of a solid pharmaceutical composition comprising rivaroxaban, or a pharmaceutically acceptable salt or solvate thereof, the process comprising
(a) providing a mixture comprising rivaroxaban, or a pharmaceutically acceptable salt or solvate thereof, and at least one pharmaceutically acceptable excipient; and
(b) converting said mixture to a solid pharmaceutical composition.

It is preferred that the process according to the invention is performed under dry conditions. The term "dry conditions" as used herein refers to a process involving less than 10 %, particularly less than 5 %, and most preferably less than 1 % of water or organic solvents by weight of the solid components used in the process. It is particularly preferred that the process according to the invention is carried out without using any water or organic solvents.

It is also preferred that the process is performed at a temperature well below the melting temperature of rivaroxaban. In particular, the process is performed at a temperature of not more than 200 °C, preferably not more than 150 °C, more preferably not more than 100 °C, and most preferably not more than 80 °C.

The process according to the invention preferably comprises single-stage or multi-stage micronization of rivaroxaban to obtain rivaroxaban in micronized form with d₉₀ < 60 µm, preferably d₉₀ < 40 µm, and more preferably d₉₀ < 30 µm. Preferably, micronization of rivaroxaban is performed in the dry state using dry mills, such as cutting mills, pin/cage mills, hammer mills, jet mills, fluidized bed jet mills, ball mills and roller mills. It is particularly preferred that micronized rivaroxaban is used in step (a).

According to one embodiment, step (a) comprises dry mixing rivaroxaban with at least one excipient.

According to another embodiment, step (a) comprises dry granulation of rivaroxaban with at least one excipient and optionally mixing the obtained granulate with additional excipients. Dry granulation can be performed for instance using roller or slugging compaction and subsequently milling the obtained compact into granules.

According to another embodiment, step (a) comprises co-milling of rivaroxaban with at least one excipient and optionally mixing the obtained co-milled mixture with additional excipients. Preferably, the rivaroxaban is co-milled with at least one hydrophilic excipient. Suitable hydrophilic excipients include monosaccharides and oligosaccharides such as glucose, fructose, saccharose, lactose (anhydrous and monohydrate), raffinose, trehalose and dextrates and sugar alcohols such as mannitol, sorbitol, maltitol, xylitol and lactitol. Sugar alcohols such as mannitol or sorbitol are particularly preferred. Co-milling can be performed for instance using a jet mill. The co-milled mixture can further be micronized as described above.

In another aspect of the invention rivaroxaban can be co-milled with disintegrant and/or surfactant, such as copovidon and/or sodium dodecyl sulphate, sucrose palmitate or poloxamer.

In another embodiment of the present invention rivaroxaban is
1) i) micronized or
   ii) co-micronized with at least one excipient or
   iii) co-milled or co-grinded with at least one excipient,
2) the micronized or milled product obtained by 1 i), 1 ii) or 1 iii) is further mixed with at least one excipient, and
3) the product obtained by 2) is agglomerated or granulated by wet granulation without preparing a solution or suspension of rivaroxaban in the granulation liquid.

The average particle size of the agglomerated or granulated particles can be in the range of 20 to 300 µm, preferably 30 to 200 µm. The agglomeration and granulation is used to improve physical properties, such as flowability and/or compressibility of the product.

According to another embodiment, step (a) comprises hot melt granulation of rivaroxaban with at least one excipient and optionally mixing the obtained granulate with additional excipients. Hot melt granulation can be performed by granulating rivaroxaban with at least one excipient in a granulating machine such as a high-shear mixer at a temperature of at least the melting point of at least one excipient. Preferably, rivaroxaban is granulated with at least one low-melting binder having a melting point below 120 °C, particularly below 100 °C, and most preferably below 80 °C, at a temperature of at least the melting point of said binder. Preferred low-melting binders are those defined above for the first aspect of the invention.

Hot melt granulation can also be performed by heating at least one excipient to at least its melting point, dispersing rivaroxaban and optionally further excipients in the molten excipient and spraying the dispersion onto at least one further excipient. Preferably, the molten excipient is a low-melting binder as described above. In particular, rivaroxaban and a surfactant can be dispersed in molten binder and the dispersion sprayed onto a mixture of filler and optionally disintegrant. The obtained mixture is typically cooled, milled into granules and sieved.

According to another embodiment, step (a) comprises hot melt extrusion of rivaroxaban with at least one excipient, milling the extrudate and optionally mixing the milled extrudate with additional excipients. Extrusion can be performed using conventional extruders. The obtained extrudate is milled for instance using a jet mill. Preferably, rivaroxaban is extruded with at least one low-melting binder having a melting point below 120 °C, particularly below 100 °C, most preferably below 80 °C, at a temperature of at least the melting point of said binder. Preferred low-melting binders are those defined above for the first aspect of the invention.

In step (b), the mixture is preferably compressed to give a tablet. The obtained tablet is optionally film-coated according to coating methods generally known in the art.

It has surprisingly been found that tablets prepared according to the above process exhibit a dissolution profile and bioavailability that is at least comparable to that of known rivaroxaban formulations while offering important advantages in terms of process economy and suitability for industrial application. In particular, processes performed under dry conditions are substantially less complex and expensive than processes involving for instance moist granulation and/or spray-drying. Moreover, processes using hot melt granulation or hot melt extrusion at the relatively low temperatures defined above surprisingly allow for the preparation of tablets having good dissolution and bioavailability properties without subjecting the active agent to temperatures leading to any substantial decomposition thereof.

Further preferred embodiments of the solid pharmaceutical composition with regard to specific components and their amounts as well as with regard to dissolution properties and bioavailability are as described above for the first aspect of the invention.

The invention also relates to a solid pharmaceutical composition prepared by the process according to the invention.

The invention will be further illustrated by way of the following examples.

### Examples

### Example 1

### Single-stage micronization of rivaroxaban

Rivaroxaban was micronized using single-stage milling. A fluid jet mill with Venturi inlet nozzle operating at a pressure of about 12 bar, a ring pressure of about 11 bar and a dosing mass flow of 20-30 g/min was used (Jetmill MC50, Jet Pharma S.A.). The resultant rivaroxaban was determined to have a particle size distribution of d₉₀ < 15 µm.

The specific surface area of the rivaroxaban was measured by a gas sorption system based on the nitrogen adsorption, using the 6-point Brunauer, Emmett and Teller (BET) method. The measurement showed a change from 0.4 m²/g before milling to 7 m²/g after micronization.

### Example 2

### Multi-stage micronization of rivaroxaban

Rivaroxaban was micronized using multi-stage milling. A fluid jet mill with Venturi inlet nozzle operating at a pressure of about 6 bar, a ring pressure of about 4 bar and a dosing mass flow of 20-30 g/min was used in the first stage (Jetmill MC50, Jet Pharma S.A.). The resultant rivaroxaban was determined to have a particle size distribution of d₉₀ < 50 µm.

Subsequently, the rivaroxaban was micronised in a second stage with the Venturi inlet nozzle operating at a pressure of about 12 bar, a ring pressure of about 11 bar and a dosing mass flow of 20-30 g/min (Jetmill MC50, Jet Pharma S.A.). The resultant rivaroxaban was determined to have a particle size distribution of d₉₀ < 10 µm.

### Example 3

### Co-milling of rivaroxaban together with hydrophilic excipient

50 g of rivaroxaban and 100 g of sorbitol were co-milled using an air-jet mill with an inlet air pressure of about 9 bar and a milling pressure of about 8.5 bar. The resultant mixture of co-milled rivaroxaban and hydrophilic excipient was micronized as described above in example 1 or 2.

### Example 4

### Preparation of tablets by direct compression

**Tablet compositions were prepared as follows:**

| | **4A** | **4B** | **4C** | **4D** | **4E** | **4F** | **4G** |
|---|---|---|---|---|---|---|---|
| Rivaroxaban | 10.0 | 10.0 | 10.0 | 10.0 | 10.0 | 10.0 | 10.0 |
| Sodium lauryl sulfate | 2.0 | 2.0 | 2.0 | 2.0 | 2.0 | 2.5 | 2.5 |
| Mannitol | 42.5 | 42.5 | | | | | |
| Lactose spray-dried | | | 42.5 | | 38.5 | 43.9 | 43.9 |
| Cellulose, microcrystalline | 30.0 | | | | 30.0 | 40.0 | 30.0 |
| Calcium hydrogen phosphate | | 30.0 | 30.0 | 68.5 | | | |
| Citric acid | | | | | | | 10.0 |
| Crospovidone | | | | 4.0 | | | |
| Croscarmellose | | | | | 4.0 | 3.0 | 3.0 |
| Magnesium stearate | 0.5 | 0.5 | 0.5 | 0.5 | 0.5 | 0.6 | 0.6 |
| Total mass (mg) | 85.0 | 85.0 | 85.0 | 85.0 | 85.0 | 100.0 | 100.0 |

Micronized rivaroxaban according to example 1 was dry-mixed with the other excipients. The resulting mixture was directly compressed to form tablets.

### Example 5

### Preparation of tablets by dry granulation

**Tablet compositions were prepared as follows:**

| | **5A** | **5B** | **5C** | **5D** | **5E** |
|---|---|---|---|---|---|
| Rivaroxaban | 10.0 | 10.0 | 10.0 | 10.0 | 10.0 |
| Sodium lauryl sulfate | 2.0 | 2.0 | 2.0 | 2.0 | 2.0 |
| Mannitol | 42.5 | 42.5 | | | |
| Lactose spray-dried | | | 42.5 | | 38.5 |
| Cellulose, microcrystalline | 30.0 | | | | 30.0 |
| Calcium hydrogen phosphate | | 30.0 | 30.0 | 68.5 | |
| Crospovidone | | | | 4.0 | |
| Croscarmellose | | | | | 4.0 |
| Magnesium stearate | 0.5 | 0.5 | 0.5 | 0.5 | 0.5 |
| Total mass (mg) | 85.0 | 85.0 | 85.0 | 85.0 | 85.0 |

Rivaroxaban was mixed with the other excipients, except Magnesium stearate, sieved and mixed again. The mixture was dry granulated with a roller-compactor using a Fitzpatrik laboratory machine. The resulting compact was milled into granules, mixed with Magnesium stearate and compressed into tablets.

### Example 6

### Preparation of tablets by hot melt granulation

**Tablet compositions were prepared as follows:**

| | **6A** | **6B** | **6C** |
|---|---|---|---|
| Rivaroxaban | 10.0 | 10.0 | 10.0 |
| PEG 6000 | 15.0 | | |
| Poloxamer 188 | | 15.0 | 15.0 |
| Mannitol | 41.5 | 43.5 | |
| Maize starch | | | 43.5 |
| Crospovidone | 8.0 | 6.0 | 6.0 |
| Cellulose, microcrystalline | 10.0 | 10.0 | 10.0 |
| Colloidal silicon dioxide | 0.5 | 0.5 | 0.5 |
| Total mass (mg) | 85.0 | 85.0 | 85.0 |

Rivaroxaban was mixed with the other excipients (except colloidal silicon dioxide) in a high-shear granulation machine at a temperature of about 55-70 °C for about 5 to 15 minutes. The mixture was cooled down to ambient temperature and mixed with colloidal silicon dioxide. The resulting mixture was compressed into tablets.

### Example 7

### Preparation of tablets by granulation with melted binder

**Tablet compositions were prepared as follows:**

| | **7A** | **7B** |
|---|---|---|
| | | |
| Rivaroxaban | 10.0 | 10.0 |
| Gelucire^{®} 50/14 | 10.0 | |
| Poloxamer 188 | | 10.0 |
| Mannitol (Pearlitol 200 SD) | 40.0 | 40.0 |
| Sodium lauryl sulfate | 1.0 | 1.0 |
| Crospovidone | 2.0 | 1.5 |
| | | |
| Avicel PH 200 | 15.0 | 15.0 |
| Mannitol (Pearlitol 200 SD) | 13.5 | 15.5 |
| Crospovidone | 6.0 | 4.5 |
| Aerosil 200 | 1.0 | 1.0 |
| Magnesium stearate | 1.5 | 1.5 |
| | | |
| Total mass (mg) | 100.0 | 100.0 |

Rivaroxaban and sodium lauryl sulfate were dispersed in the melted binder (Gelucire^{®} or Poloxamer). The dispersion was sprayed onto a mixture of a first amount of mannitol (Pearlitol 200 SD) and crospovidone using adapted laboratory fluid bed equipment Glatt GPCG3. The obtained dispersion was cooled, milled and sieved through a 1 mm sieve. The sieved dispersion was mixed with Avicel and the remaining amounts of mannitol and crospovidone and finally with Aerosil and magnesium stearate and then compressed into tablets.

## Claims

1. Solid pharmaceutical composition comprising rivaroxaban, or a pharmaceutically acceptable salt or solvate thereof, and at least one pharmaceutically acceptable excipient.

2. The composition according to claim 1, which is in the form of a tablet or capsule or in the form of pellets or granules.

3. The composition according to claim 1 or 2, which comprises at least 35 wt.-%, particularly 40 to 70 wt.-%, more preferably 40 to 60 wt.-%, and most preferably 45 to 55 wt.-% of a water-soluble filler.

4. The composition according to any one of claims 1 to 3, which comprises a water-soluble filler and a water-insoluble filler in a weight ratio of 1:1 to 10:1, particularly 1:1 to 5:1, and most preferably 1.1:1 to 1.5:1.

5. The composition according to claim 3 or 4, wherein the water-soluble filler is selected from lactose, mannitol, sorbitol, xylitol and mixtures thereof.

6. The composition according to claim 4 or 5, wherein the water-insoluble filler is selected from microcrystalline cellulose, powdered cellulose, siliconized microcrystalline cellulose, calcium hydrogen phosphate, calcium carbonate, calcium lactate and mixtures thereof.

7. The composition according to any one of claims 1 to 6, which comprises 0.5 to 30 wt.-%, particularly 1 to 10 wt.-%, more preferably 1.5 to 5 wt.-%, and most preferably 2 to 3 wt.-% of surfactant.

8. The composition according to any one of claims 1 to 7, which comprises 1 to 20 wt.-%, particularly 2 to 10 wt.-%, more preferably 3 to 8 wt.-%, and most preferably 4 to 7 wt.-% of disintegrant.

9. The composition according to any one of claims 1 to 8, which comprises at least one binder having a melting point below 120 °C, particularly below 100 °C, and most preferably below 80 °C.

10. The composition according to any one of claims 1 to 9, which comprises
(a) 0.1 to 60 wt.-%, particularly 0.5 to 40 wt.-%, more preferably 1 to 20 wt.-%, and most preferably 5 to 15 wt.-% of rivaroxaban or a pharmaceutically acceptable salt or solvate thereof;
(b) 30 to 99 wt.-%, particularly 50 to 95 wt.-%, more preferably 70 to 90 wt.-%, and most preferably 80 to 87 wt.-% of filler;
(c) 0 to 40 wt.-%, particularly 1 to 20 wt.-%, more preferably 2 to 10 wt.-%, and most preferably 3 to 7 wt.-% of binder;
(d) 0 to 20 wt.-%, particularly 2 to 10 wt.-%, more preferably 3 to 8 wt.-%, and most preferably 4 to 7 wt.-% of disintegrant;
(e) 0 to 30 wt.-%, particularly 1 to 10 wt.-%, more preferably 1.5 to 5 wt.-%, and most preferably 2 to 3 wt.-% of surfactant;
(f) 0 to 10 wt.-%, particularly 0.25 to 5 wt.-%, and more preferably 0.5 to 2 wt.-% of lubricant;
(g) 0 to 10 wt.-%, particularly 0.25 to 5 wt.-%, and more preferably 0.5 to 2 wt.-% of glidant;
(h) 0 to 1 wt.-%, particularly 0.01 to 0.5 wt.-%, and more preferably 0.05 to 0.2 wt.-% of coloring agent; and
(i) 0 to 15 wt.-%, particularly 1 to 12 wt.-% of organic acid.

11. The composition according to any one of claims 1 to 10, which exhibits a release profile **characterized in that** at least 40 %, particularly at least 50 %, more preferably at least 60 %, and most preferably at least 70 % by weight of the active agent are released within a period of 30 minutes under non-sink conditions (USP paddle 75 ppm, 900 ml acetate buffer pH 4.5).

12. The composition according to any one of claims 1 to 11, which is obtained by co-milling of the rivaroxaban and the at least one pharmaceutical acceptable excipient.

13. A process for the preparation of a solid pharmaceutical composition comprising rivaroxaban, or a pharmaceutically acceptable salt or solvate thereof, the process comprising
(a) providing a mixture comprising rivaroxaban, or a pharmaceutically acceptable salt or solvate thereof, and at least one pharmaceutically acceptable excipient; and
(b) converting said mixture to a solid pharmaceutical composition.

14. The process according to claim 13, which is carried out without using water or organic solvents.

15. The process according to claim 13 or 14, which is performed at a temperature of not more than 200 °C, preferably not more than 150 °C, more preferably not more than 100 °C, and most preferably not more than 80 °C.

16. The process according to any one of claims 13 to 15, wherein step (a) comprises
(i) dry granulation of rivaroxaban with at least one excipient and optionally mixing the obtained granulate with additional excipients; or
(ii) co-milling of rivaroxaban with at least one excipient and optionally mixing the obtained co-milled mixture with additional excipients; or
(iii) hot melt granulation of rivaroxaban with at least one excipient and optionally mixing the obtained granulate with additional excipients; or
(iv) hot melt extrusion of rivaroxaban with at least one excipient, milling the extrudate and optionally mixing the milled extrudate with additional excipients.

17. A solid pharmaceutical composition prepared by the process according to any one of claims 13 to 16.
